# FASCICULE DE BREVET EUROPEEN

(11) **EP 0 797 589 B1**
(45) Date de publication et mention de la délivrance du brevet: **28.12.2005**
(21) Numéro de dépôt: 96933490.3
(22) Date de dépôt: 04.10.1996
(51) Int. Cl.: C07K 14/47

(54) **PEPTIDES UTILISABLES COMME VECTEURS POUR L'ADRESSAGE INTRACELLULAIRE DE MOLECULES ACTIVES**
PEPTIDE ALS VEKTOREN ZUR INTRAZELLULÄREN ADRESSIERUNG VON AKTIVEN MOLEKÜLEN
PEPTIDES USABLE AS VECTORS FOR THE INTRACELLULAR ADDRESSING OF ACTIVE MOLECULES

(30) Priorité: 05.10.1995 FR 9511714
(43) Date de publication de la demande: 01.10.1997
(73) Titulaire: CENTRE NATIONAL DE LA RECHERCHE SCIENTIFIQUE, 75016 Paris Cédex (FR)
(72) Inventeur: CHASSAING, Gérard, F-75013 Paris (FR); PROCHIANTZ, Alain, F-75006 Paris (FR)
(74) Mandataire: Vialle-Presles, Marie José
(86) Numéro de dépôt international: PCT/FR1996/001553
(87) Numéro de publication internationale: WO 1997/012912

(56) Documents cités:
- JOURNAL OF BIOLOGICAL CHEMISTRY , vol. 269, no. 14, 8 Avril 1994, MD US, pages 10444-10450, XP002006023 D DEROSSI ET AL.: "The third helix of the Antennapedia homeodomain translocates through biological membranes" cité dans la demande
- PROCEEDINGS OF THE NATIONAL ACADEMY OF SCIENCES OF USA, vol. 90, no. 19, 1 Octobre 1993, WASHINGTON US, pages 9120-9124, XP002006024 I LE ROUX ET AL.: "Neurotrophic activity of the Antennapedia homeodomain depends on its specific DNA-binding properties" cité dans la demande
- JOURNAL OF BIOLOGICAL CHEMISTRY, vol. 271, no. 30, 26 Juillet 1996, MD US, pages 18188-18193, XP002024485 D DEROSSI ET AL.: "Cell internalization of the third helix of the Antennapedia homeodomain is receptor-independent"

## Description

La présente Invention est relative à une classe de peptides capables de traverser les membranes cellulaires et de parvenir aux divers compartiments de la cellule.

Le problème de l'entrée dans les cellules vivantes de différentes substances dotées de propriétés pharmacologiques, et de leur accès aux divers compartiments intracellulaires, en particulier le compartiment cytoplasmique et le compartiment nucléaire, revêt une grande importance, tant pour la recherche que pour l'utilisation thérapeutique.

On connaît actuellement un nombre limité de moyens d'introduire des substances telles que les polypeptides et les oligonucléotides dans les compartiments intracellulaires. Parmi les différentes techniques actuellement proposées on peut citer :
1. La transfection de gènes, et les techniques dérivées permettant d'améliorer *in vivo* ou *in vitro* les taux de transfection, telles que la précipitation au phosphate de calcium, l'utilisation de lipides cationiques, l'électroporation, la trituration (scrape loading), l'utilisation de vecteurs viraux, etc...
2. La liaison à des récepteurs de la membrane cellulaire ; ces récepteurs sont par la suite endocytosés, et libèrent dans le compartiment cytoplasmique les molécules liées. Dans cette catégorie on peut citer le récepteur du folate, la toxine diphtérique, ou des facteurs de transcription comme la protéine TAT du rétrovirus HIV. Le mécanisme de transport impliquant ces récepteurs est encore mal connu, mais nécessite dans tous les cas une étape d'endocytose.
3. Les peptides de type homéodomaine. Des travaux antérieurs effectués par l'équipe des Inventeurs sur l'homéodomaine du facteur de transcription Antennapedia (AntpHD) ont permis de montrer que les peptides homéodomaine traversent les membranes plasmiques par un processus énergie-indépendant, et donc distinct de l'endocytose. La 3ème hélice du peptide homéodomaine possède les mêmes propriétés [JOLIOT et al. Proc. Natl. Acad. Sci., USA, 88, P. 1864-1868 (1991) ; DEROSSI et. al., J. Biol. Chem. 269, 14, p. 10444-10450, (1994)].

Ces propriétés ont été utilisées pour internaliser dans des cellules des polypeptides et des oligonucléotides liés à l'homéodomaine ou à l'hélice 3, [PEREZ et al., J. Cell. Science, 102, p. 712-722, (1992)] par fusion génétique ou liaison biochimique. Cette entrée est quantitative, et le vecteur et son chargement sont retrouvés dans 100% des cellules ; en outre, l'internalisation est indépendante du type cellulaire concerné.

Le plus petit fragment de l'homéodomaine capable de traverser les membranes et de servir de vecteur à d'autres peptides ou bien à des oligonucléotides est un peptide de 16 acides aminés, correspondant à l'hélice 3. Ce peptide, qui comprend les acides aminés 43 à 58 de l'homéodomaine, est dénommé ci-après. A titre d'exemple, la séquence du peptide 43-58 de l'homéodomaine Antp est la suivante :

Arg-Gln-Ile-Lys-Ile-Trp-Phe-Gln-Asn-Arg-Arg-Met-Lys-Trp-Lys-Lys

Cette séquence est représentée dans la liste de séquences en annexe sous le numéro SEQ ID N0:1.

Le mécanisme par lequel ce peptide pouvait pénétrer dans les cellules vivantes a fait l'objet de diverses études, et il était supposé jusqu'à présent que sa structure en alpha-hélice était indispensable pour l'internalisation. L'équipe des Inventeurs a montré précédemment que certaines substitutions ou délétions dans la séquence peptidique, qui modifiaient la structure du peptide, interféraient avec l'activité de celui-ci. Par exemple, un peptide dans lequel les 2 Trp (48 et 56) sont remplacés par deux Phe, ou un peptide comprenant les acides aminés 41 à 55 de l'homéodomaine ne sont pas internalisés (DEROSSI et al., 1994, publication précitée). Une autre équipe [BRUGIDOU et al., Biophys. Biochem. Res. Com., 214:2, pp 685-693, (1995)] a observé l'internalisation de peptides constituant des analogues structuraux du peptide 43-58 ; pour cela, ils ont construit, à partir d'un peptide 43-58 (différant du peptide 43-58 de l'homéodomaine Antp en ce que les 2 isoleucines aux positions 45 et 47 sont remplacées par des valines), des variants de type *retro-inverso.* Les variants *retro-inverso*, qui permettent d'imiter la structure tridimensionnelle de peptides naturels, sont constitués par des acides aminés de la série D (au lieu des acides aminés de la série L des peptides naturels) enchaînés selon une séquence inverse de celle du peptide à reproduire.

Les Inventeurs ont maintenant cherché à définir les caractéristiques minimales des séquences d'acides aminés capables de servir de vecteur d'internalisation et d'adressage de polypeptides et d'oligonucléotides, et dans ce but ont synthétisé plusieurs peptides en modifiant spécifiquement certains résidus.

La présente Invention a pour objet un peptide capable de pénétrer dans une cellule vivante, défini par l'une des séquences (I) ou (Ia) suivantes :
NH₂

X₁-X₂-X₃-X₄-X₅-X₆-X₇-X₈-X₉-X₁₀-X₁₁-X₁₂-X₁₃X₁₄-X₁₅-X₁₆ (I)

X₁₆-X₁₅-X₁₄-X₁₃-X₁₂-X₁₁-X₁₀-X₉-X₈-X₇-X₆-X₅-X₄-X₃-X₂-X₁ (Ia)

COOH où X₁, X₂, X₃, X₄, X₅, X₆, X₇, X₈, X₉, X₁₀, X₁₁, X₁₂, X₁₃, X₁₄, X₁₅, X₁₆ représentent chacun un acide α-aminé, lequel peptide est caractérisé en ce que :
- il comprend 6 ou 7 acides aminés hydrophobes ;
- X₆ représente le tryptophane ;
- X₃, X₇, et X₁₄, représentent des acides aminés hydrophobes ;
- X₁, X₂, X₄, et X₉ représentent des acides aminés non-hydrophobes ;
- X₁₅ et X₁₆ représentent l'arginine ou la lysine ;
à l'exception des peptides suivants :
- le peptide dont la séquence est représentée dans la liste de séquences en annexe sous le numéro SEQ ID NO:1 ;
- les peptides dans lesquels X₃ et X₅ représentent chacun un résidu valine.

Selon un mode de réalisation préféré d'un peptide conforme à l'invention, il comprend 6 acides aminés hydrophobes et 10 acides aminés non-hydrophobes.

Les acides aminés hydrophobes sont l'alanine, la valine, la leucine, l'isoleucine, la proline, la phénylalanine, le tryptophane, et la méthionine.

Les acides aminés non-hydrophobes peuvent être des acides aminés polaires (glycine, sérine, thréonine, cystéine, tyrosine, asparagine, glutamine), acides (acide aspartique ou glutamique) ou basiques (lysine, arginine ou histidine), ou une association d'acides aminés de ces trois catégories.

L'enchaînement d'acides aminés non-hydrophobes polaires ou chargés, et d'acides aminés hydrophobes confère aux peptides conformes à l'invention un caractère amphiphile, qui d'après les expérimentations effectuées par les Inventeurs, apparaît essentiel pour leurs propriétés. Ces expérimentations ont en outre permis de mettre en lumière d'autres caractéristiques essentielles pour la translocation intracellulaire, et de montrer que :
- la translocation intracellulaire ne nécessite pas de récepteur spécifique, et peut donc concerner tous les types cellulaires ;
- la structure en alpha-hélice n'intervient pas dans la translocation intracellulaire, (mais joue sans doute un rôle dans l'adressage nucléaire) ;
- les propriétés amphiphiles du peptide, ainsi que la présence d'un résidu Trp apparaissent en revanche importantes pour la translocation.

Selon un autre mode de réalisation d'un peptide conforme à l'invention, X₁₄ représente le tryptophane ou l'isoleucine.

Les propriétés de pénétration intracellulaire des peptides conformes à l'invention sont comparables à celles de l'hélice 3 d'un peptide homéodomaine, et permettent leur utilisation comme vecteur d'internalisation et d'adressage intra-cellulaire, pour introduire dans des cellules vivantes des molécules actives sur les fonctions cellulaires, en particulier d'autres peptides, ou des séquences nucléotidiques.

Pour obtenir un adressage plus spécifiquement cytoplasmique de la molécule active que l'on souhaite introduire, on utilisera de préférence un peptide conforme à l'invention dans lequel au moins l'un des acides aminés en position X₃, X₇, et X₁₄ est une proline.

Pour la mise en oeuvre de la présente Invention, le polypeptide ou l'oligonucléotide à transporter est lié à un peptide conforme à l'invention.
Les produits de fusion d'un peptide conforme à l'invention avec une autre séquence peptidique, ou avec une séquence oligonucléotidique peuvent être obtenus par différents moyens connus en eux-mêmes. Dans le cas d'un polypeptide on pourra utiliser par exemple par les techniques classiques du génie génétique, ou de la synthèse peptidique. Dans le cas d'un oligonucléotide on utilisera par exemple la technique décrite par LEMAITRE et al.[Proc. Natl. Acad. Sci. USA, 84, 648-652, (1987)], ou celle de MATSUEDA et al. [Chemistry Letters, p. 951-952, (1978) and Int. J. of Peptide and Protein Research, p. 107-112 (1986)].

La présente Invention sera mieux comprise à l'aide du complément de description qui va suivre, qui se réfère à un exemple montrant les propriétés de différents peptides conformes à l'Invention.

Il doit être bien entendu toutefois que cet exemple est donné uniquement à titre d'illustration de l'objet de l'Invention dont il ne constitue en aucune manière une limitation.

### EXEMPLE

10 peptides, dont les séquences sont représentées dans la liste de séquences en annexe sous les numéros SEQ ID N0:1 à SEQ ID N0:10 ont été synthétisés. Tous ces peptides ont en outre été pourvus à leur extrémité N-terminale d'un bras aminopentanoique et d'une biotine permettant de suivre leur internalisation ; les peptides ainsi modifiés sont représentés sur les Tableaux I et II ci-après.

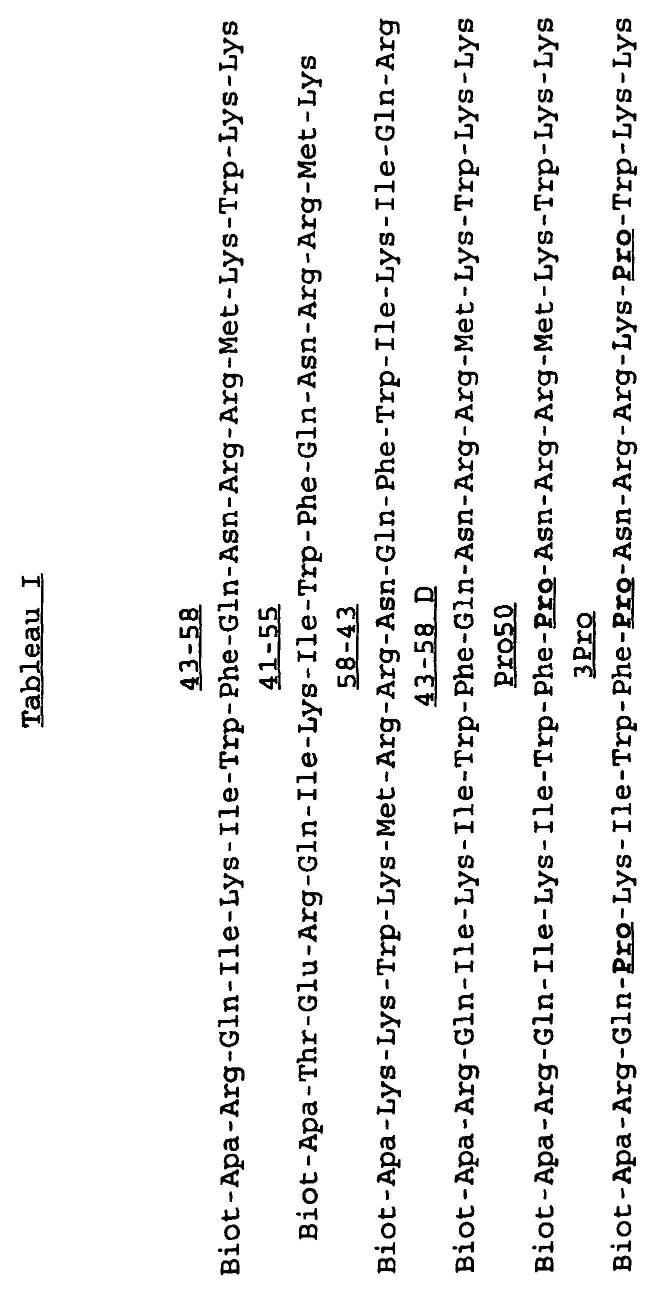

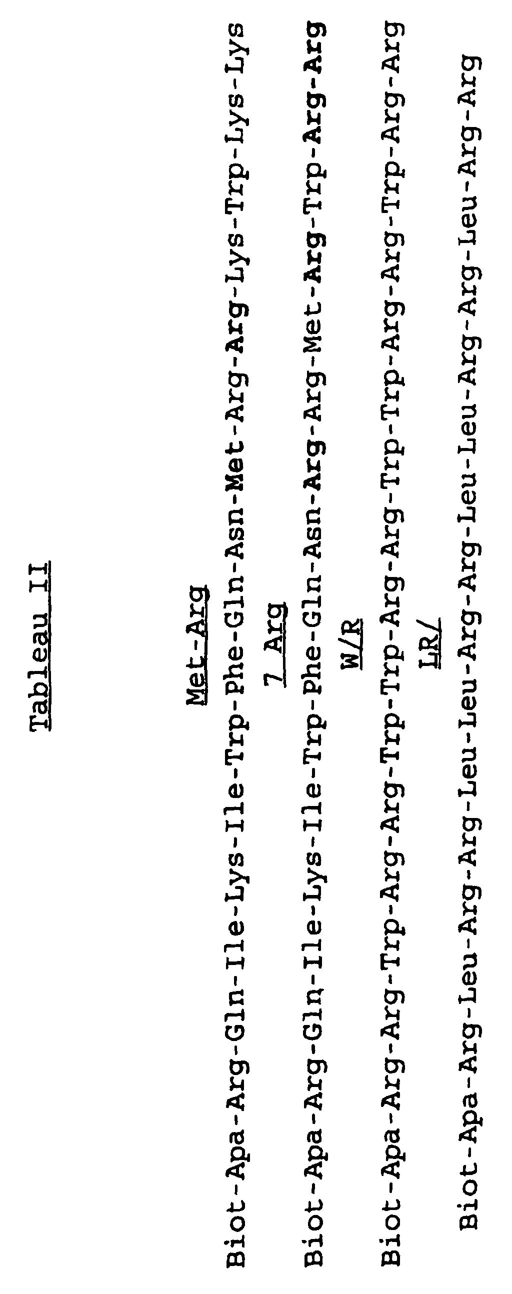

### Légende du Tableau I

43-58 : Peptide hélice 3°
41-55 : Témoin : Peptide non-internalisé (voir DEROSSI et al., 1994)
58-43 : Séquence inverse
43-58 D : Peptide 3° hélice (43-58) constitué d'acides aminés de la série D
Pro50 : Peptide 43-58 avec une Proline en 50 (au lieu de Gln)
3Pro : Peptide 43-58 avec 3 Prolines en positions 45, 50 et 55, à la place respectivement des résidus Ile, Gln et Lys.

### Légende du Tableau II

Met-Arg : Peptide 43-58 avec Met en 52 (au lieu de Arg) et Arg en 54 (au lieu de Met)
7Arg : Toutes les Lysines du peptide 43-58, sauf celle en 46 sont remplacées par des Arg
W/R : Peptide 43-58 très modifié, consiste essentiellement en une succession de Trp et Arg
L/R : Peptide W/R avec des Leu en place des Trp.

L'entrée de ces différents peptides dans des cellules en culture a été étudiée, dans les mêmes conditions et en utilisant les mêmes protocoles que ceux décrits par DEROSSI et al. (1994, publication précitée).

L'entrée des peptides dans les cellules nerveuses ou des fibroblastes en culture a été examinée à 4 et 37°C.

L'entrée des peptides du Tableau I a été testée par microscopie confocale, gel d'électrophorèse et ELISA.

L'entrée des peptides du Tableau II a été testée uniquement par microscopie confocale ; en effet, l'absence de lysine dans ces peptides rend les fixations très difficiles et demande donc une observation rapide incompatible avec des transferts sur filtres ou des tests ELISA (multiples lavages).

### 1) Internalisation à 37°C et à 4°C des peptides 43-58,58-43, D43-58, Pro50 et 3Pro

Des cellules de cortex embryonnaire E15 ont été incubées (1,1.10 cellules/ml) pendant 2h à 37°C ou à 4°C, avec les peptides 43-58, 58-43, D43-58, Pro50 et 3Pro, à la concentration de 44 µM pour une quantité 1X, ou sans peptide (puits C).

La présence des peptides dans le milieu d'incubation et dans les cellules après lavage de celles-ci à la trypsine a été analysée sur gel d'électrophorèse SDS-PAGE 12-22% et par électrotransfert.

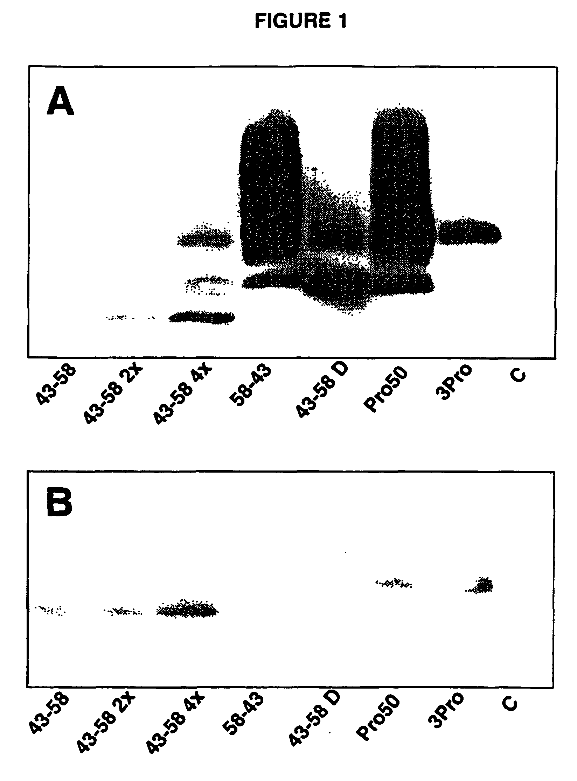

Les résultats sont illustrés sur les figures 1A, 1B (37°C) et 2 (4°C).

Légende de la figure 1 :
A : autoradiogramme des extraits cellulaires après révélation par bioluminescence (LUMINOL®, AMERSHAM).
B : autoradiogramme des milieux d'incubation après révélation par bioluminescence.

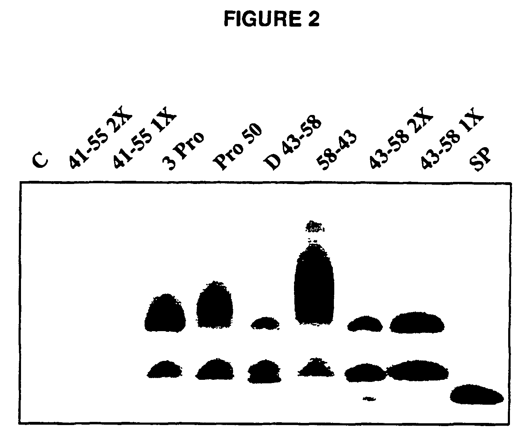

Légende de la figure 2 :
Le puits SP correspond au dépôt sur le gel de 1 µl de substance P.
Tous les peptides du tableau 1, à l'exception de 41-55 sont internalisés à 4 et 37°C et récupérés dans les cellules. Le peptide 43-58 est dégradé (50% environ) à 37°C mais pas à 4°C.

### 2) Localisation cellulaire des peptides 43-58, 51-55, 58-43, D43-58, Pro50 et 3Pro à 37°C et 4°C.

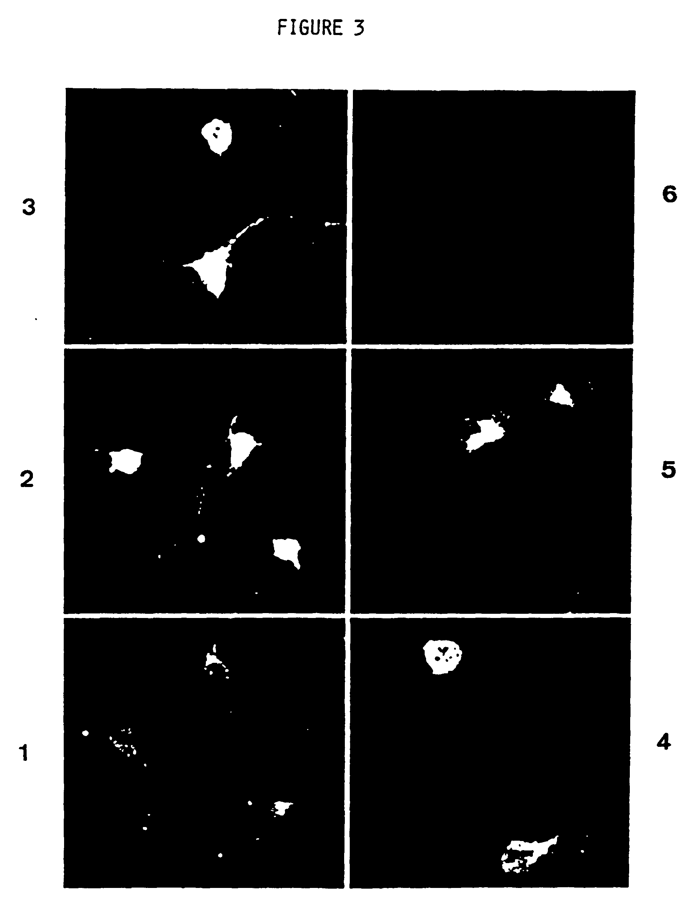

Les cellules de cortex et striatum E15 sont mises en culture sur des lamelles de verre à une densité de 25000 cellules/cm² pendant 2 jours. Les peptides sont tous ajoutés à une concentration de 20 µM finale. Après 2h d'incubation à 37°C, les cellules sont lavées, fixées et la présence de biotine est révélée par streptavidine fluorescente. Les sections observées en microscopie confocale sont présentées sur les figures 3 (37°C) et 4 (4°C). Les cellules ayant intégré le peptide apparaissent fluorescentes, sur fond noir.

Légende de la figure 3 :

| | | | |
|---|---|---|---|
| 1 | peptide 43-58, | 2 | peptide 58-43, |
| 3 | peptide D43-58, | 4 | peptide Pro50 |
| 5 | peptide 3Pro, | 6 | peptide 41-55. |

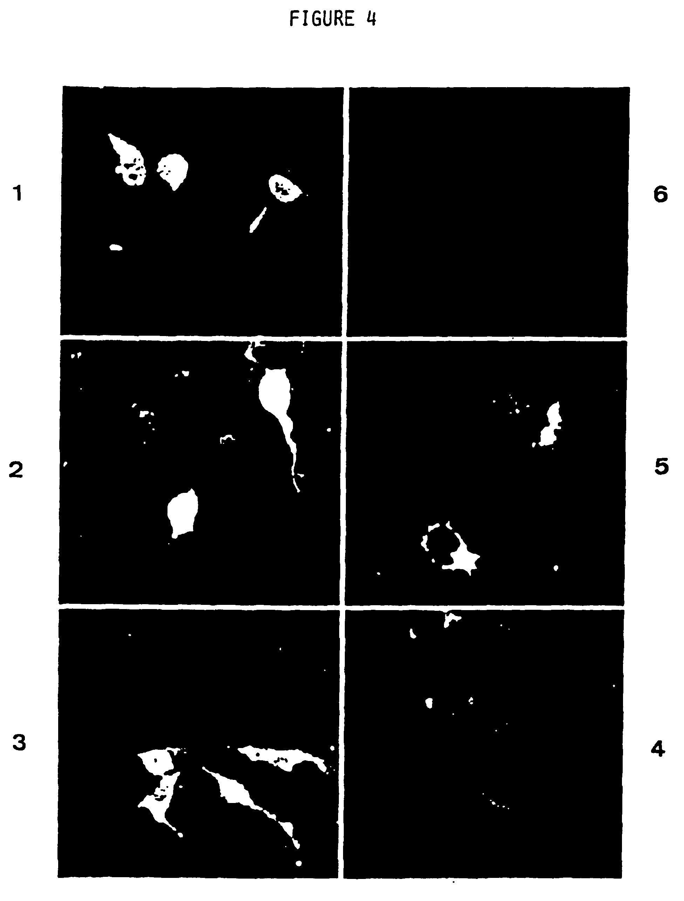

Légende de la figure 4 :

| | | | |
|---|---|---|---|
| 1 | peptide 43-58, | 2 | peptide 58-43, |
| 3 | peptide D43-58, | 4 | peptide Pro50 |
| 5 | peptide 3Pro, | 6 | peptide 41-55. |

La présence de prolines (Pro 50 ou 3Pro) n'empêche pas l'internalisation mais semble interférer avec l'adressage nucléaire. L'entrée des peptides testés est également observée dans les fibroblastes (non montré).

### 3) Dosage colorimétrique des internalisations des peptides 43-58, 51-55, 58-43, D43-58, Pro50, et 3Pro à 37°C et 4°C.

Des cellules de cortex et striatum E15 (B) ou E16 (A) ont été incubées (1,1.10⁶ cellules/ml) avec les peptides à la concentration de 17 µM (A) ou 44 µM (B) pour les quantités 1X, ou sans peptides (9). Les cellules ont été lavées au NaCl 0,5 M (A) ou à la trypsine (B). Elles sont mises en culture sur plaque ELISA, fixées et une révélation des peptides biotinylés est faite en utilisant un substrat coloré (PNPP) de la phosphatase alcaline couplée à la streptavidine.

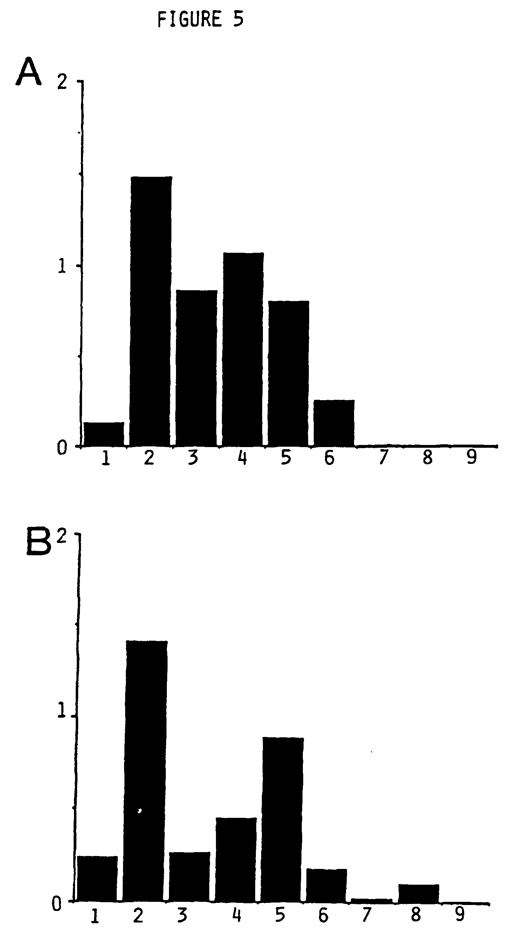

Les résultats sont illustrés par les figures 5A et 5B.

Légende des figures 5A et 5B :

| | |
|---|---|
| A | expérience à 37°C ; |
| B | expérience à 4°C |
| 1 | peptide 43-58 1X |
| 2 | peptide 43-58 4X |
| 3 | peptide 58-43 1X |
| 4 | peptide D43-58 |
| 5 | peptide Pro50 |
| 6 | peptide 3Pro |
| 7 | peptide 41-55 1X |
| 8 | peptide 41-55 4X, échantillon non présent en A |
| 9 | pas de peptide. |

### 4) Internalisation à 37°C des peptides Met-Arg, W/R et L/R

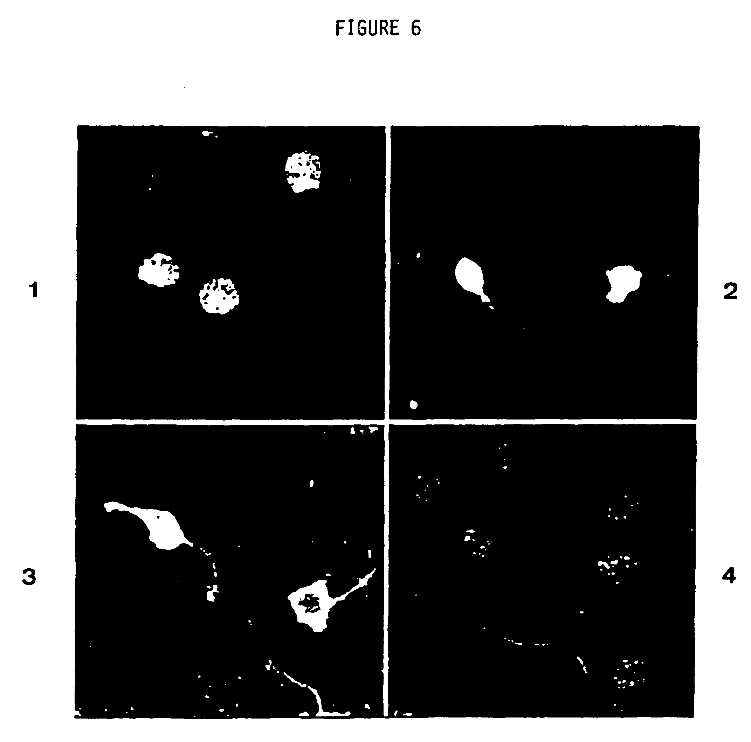

Des cellules de cortex et striatum E15 sont mises en culture sur lamelles de verre à une densité de 25000 cellules/cm² pendant 2 jours. Les peptides sont ajoutés à une concentration de 20 µM finale pour le peptide 43-58 et de 2 µM pour les autres et les cellules sont incubées 2h à 37°C. Les cellules sont lavées, fixées et la biotine des peptides est révélée par streptavidine-FITC. Les résultats sont illustrés par la Figure 6.

Légende de la figure 6 :

| | |
|---|---|
| 1 | peptide 7 Arg |
| 2 | peptide Met-Arg |
| 3 | peptide W/R |
| 4 | peptide L/R |

On observe une internalisation et un adressage cytoplasmique et nucléaire du Met-Arg, du 7Arg et du W/R. Toutefois, le peptide L/R est retrouvé dans une fraction apparemment de type vésiculaire, probablement de caractère endocytique.

### CONCLUSION

Les résultats obtenus avec les peptides du Tableau I permettent de conclure que :
- l'internalisation ne nécessite pas de récepteur puisque les peptides 58-43 et 43-58D sont internalisés. Or le premier a une séquence différente de celle du peptide initial (même si sa structure générale d'hélice amphiphile est conservée), et le peptide constitué d'acides aminés de la série D ne doit pas interagir avec un récepteur naturel composé d'acides aminés de la série L ;
- la structure en hélice alpha n'est pas nécessaire puisque l'introduction d'une proline et *a fiortiori* de 3 détruit l'hélicité. Cependant on note que l'addition des prolines peut interférer avec l'adressage nucléaire.

En ce qui concerne les peptides du Tableau II, on peut conclure des résultats obtenus que :
- l'amphiphilicité est nécessaire pour l'internalisation, mais elle n'est pas suffisante, dans la mesure où le L/R n'a pas les propriétés du W/R
- la présence et la position des résidus Trp ont leur importance.
   Les inventeurs ayant également démontré l'internalisation de l'homéodomaine de l'homéoprotéine Engrailed, qui n'a pas le résidu Trp en 56 (qui est remplacé par un résidu Ile) mais a conservé celui en 48, on peut en conclure que seul le Trp 48 est important pour la translocation.

## Revendications

1. Peptide capable de pénétrer dans une cellule vivante, défini par l'une des séquences (I) ou (Ia) suivantes :
NH₂-X₁-X₂-X₃-X₄-X₅-X₆-X₇-X₈-X₉-X₁₀-X₁₁-X₁₂-X₁₃-X₁₄-X₁₅-X₁₆-COOH (I)
NH₂-X₁₆-X₁₅-X₁₄-X₁₃-X₁₂-X₁₁-X₁₀-X₉-X₈-X₇-X₆-X₅-X₄-X₃-X₂-X₁-COOH (Ia)
où X₁, X₂, X₃, X₄, X₅, X₆, X₇, X₈, X₉, X₁₀, X₁₁, X₁₂, X₁₃, X₁₄, X₁₅, X₁₆ représentent chacun un acide α-aminé, lequel peptide est **caractérisé en ce que** :
- il comprend 6 ou 7 acides aminés hydrophobes ;
- X₆ représente le tryptophane ;
- X₃, X₇, et X₁₄, représentent des acides aminés hydrophobes ;
- X₁, X₂, X₄, et X₉ représentent des acides aminés non-hydrophobes ;
- X₁₅ et X₁₆ représentent l'arginine ou la lysine ;
à l'exception des peptides suivants :
- le peptide dont la séquence est représentée dans la liste de séquences en annexe sous le numéro SEQ ID NO:1 ;
- les peptides dans lesquels X₃ et X₅ représentent chacun un résidu valine.

2. Peptide selon la revendication 1, **caractérisé en ce qu'**il comprend 6 acides aminés hydrophobes et 10 acides aminés non-hydrophobes.

3. Peptide selon une quelconque des revendications 1 ou 2, **caractérisé en ce que** X₁₄ représente le tryptophane ou l'isoleucine.

4. Utilisation d'un peptide selon une quelconque des revendications 1 à 3, pour introduire dans des cellules vivantes une molécule active sur les fonctions cellulaires.

5. Utilisation selon la revendication 4, **caractérisée en ce que** la molécule active sur les fonctions cellulaires est un peptide.

6. Utilisation selon la revendication 4, **caractérisée en ce que** la molécule active sur les fonctions cellulaires est une séquence nucléotidique.

7. Utilisation selon une quelconque des revendications 4 à 6, **caractérisée en ce que** l'on utilise un peptide de formule I dans lequel au moins l'un des acides aminés en position X₃, X₇, et X₁₄ est une proline.

## Patentansprüche

1. Peptid, das in der Lage ist, in eine lebende Zelle einzudringen, und durch eine der folgenden Sequenzen (I) oder (Ia) definiert ist:
NH₂-X₁-X₂-X₃-X₄-X₅-X₆-X₇-X₈-X₉-X₁₀-X₁₁-X₁₂-X₁₃-X₁₄-X₁₅-X₁₆-COOH (I)
NH₂-X₁₆-X₁₅-X₁₄-X₁₃-X₁₂-X₁₁-X₁₀-X₉-X₈-X₇-X₆-X₅-X₄-X₃-X₂-X₁-COOH (Ia)
wobei X₁, X₂, X₃, X₄, X₅, X₆, X₇, X₈, X₉, X₁₀, X₁₁, X₁₂, X₁₃, X₁₄, X₁₅, X₁₆ jeweils für eine α-Aminosäure stehen, und das Peptid **dadurch gekennzeichnet ist, daß**:
- es 6 oder 7 hydrophobe Aminosäuren aufweist;
- X₆ für Tryptophan steht;
- X₃, X₇ und X₁₄ für hydrophobe Aminosäuren stehen;
- X₁, X₂, X₄ und X₉ für nicht-hydrophobe Aminosäuren stehen;
- X₁₅ und X₁₆ für Arginin oder Lysin stehen;
mit Ausnahme der folgenden Peptide:
- des Peptids, dessen Sequenz in der beigefügten Sequenzenliste unter der Nr. SEQ ID NO: 1 dargestellt ist;
- der Peptide, bei denen X₃ und X₅ jeweils für einen Valinrest stehen.

2. Peptid nach Anspruch 1, **dadurch gekennzeichnet, daß** es 6 hydrophobe Aminosäuren und 10 nicht-hydrophobe Aminosäuren aufweist.

3. Peptid nach einem der Ansprüche 1 oder 2, **dadurch gekennzeichnet, daß** X₁₄ für Tryptophan oder Isoleucin steht.

4. Verwendung eines Peptids nach einem der Ansprüche 1 bis 3 für die Einbringung eines Moleküls in lebende Zellen, auf deren Zellfunktionen es einwirkt.

5. Verwendung nach Anspruch 4, **dadurch gekennzeichnet, daß** das auf die Zellfunktionen einwirkende Molekül ein Peptid ist.

6. Verwendung nach Anspruch 4, **dadurch gekennzeichnet, daß** das auf die Zellfunktionen einwirkende Molekül eine Nukleotidsequenz ist.

7. Verwendung nach einem der Ansprüche 4 bis 6, **dadurch gekennzeichnet, daß** ein Peptid der Formel I verwendet wird, bei dem mindestens eine der Aminosäuren in Position X₃, X₇ und X₁₄ ein Prolin ist.

## Claims

1. Peptide capable of penetrating a living cell, defined by one of the following sequences, (I) or (Ia):
NH₂-X₁-X₂-X₃-X₄-X₅-X₆-X₇-X₈-X₉-X₁₀-X₁₁-X₁₂-X₁₃-X₁₄-X₁₅-X₁₆-COOH (I)
NH₂-X₁₆-X₁₅-X₁₄-X₁₃-X₁₂-X₁₁-X₁₀-X₉-X₈-X₇-X₆-X₅-X₄-X₃-X₂-X₁-COOH (Ia)
where X₁, X₂, X₃, X₄, X₅, X₆, X₇, X₈, X₉, X₁₀, X₁₁, X₁₂, X₁₃, X₁₄, X₁₅, X₁₆ each represent an α-amino acid, the said peptide being **characterised in that**:
- it comprises 6 or 7 hydrophobic amino acids;
- X₆ represents tryptophan;
- X₃, X₇ and X₁₄ represent hydrophobic amino acids;
- X₁, X₂, X₄ and X₉ represent non-hydrophobic amino acids;
- X₁₅ and X₁₆ represent arginine or lysine;
with the exception of the following peptides:
- the peptide whose sequence is represented in the list of appended sequences under the number SEQ ID N0: 1;
- the peptides in which X₃ and X₅ each represent a valine residue.

2. Peptide according to claim 1, **characterised in that** it comprises six hydrophobic amino acids and ten non-hydrophobic amino acids.

3. Peptide according to either one of claims 1 or 2, **characterised in that** X₁₄ represents tryptophan or isoleucine.

4. Use of a peptide according to any one of claims 1 to 3, for introducing into living cells a molecule that is active on the cell functions.

5. Use according to claim 4, **characterised in that** the molecule that is active on the cell functions is a peptide.

6. Use according to claim 4, **characterised in that** the molecule that is active on the cell functions is a nucleotide sequence.

7. Use according to any one of claims 4 to 6, **characterised in that** a peptide of formula I is used in which at least one of the amino acids in position X₃, X₇ and X₁₄ is a proline.


### LISTE DE SEQUENCES

(1) INFORMATIONS GENERALES:
   (i) DEPOSANT:
      (A) NOM: CENTRE NATIONAL DE LA RECHERCHE SCIENTIFIQUE - C.N.R.S.
      (B) RUE: 3, RUE MICHEL ANGE
      (C) VILLE: PARIS
      (E) PAYS: FRANCE
      (F) CODE POSTAL: 75794 CEDEX 16

      (A) NOM: CHASSAING GERARD
      (B) RUE: 11, PLACE SOUHAM
      (C) VILLE: PARIS
      (E) PAYS: FRANCE
      (F) CODE POSTAL: 75013

      (A) NOM: PROCHIANTZ ALAIN
      (B) RUE: 8, RUE MARIE-PAPE CARPENTIER
      (C) VILLE: PARIS
      (E) PAYS: FRANCE
      (F) CODE POSTAL: 75006
   (ii) TITRE DE L' INVENTION: PEPTIDES UTILISABLES COMME VECTEURS POUR L'ADRESSAGE INTRACELLULAIRE DE MOLECULES ACTIVES.
   (iii) NOMBRE DE SEQUENCES: 10
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
   (vi) DONNEES DE LA DEMANDE ANTERIEURE:
      (A) NUMERO DE LA DEMANDE: FR 95 11714
      (B) DATE DE DEPOT: 05-OCT-1995
(2) INFORMATIONS POUR LA SEQ ID NO: 1:
   (i) CARACTERISTIQUES DE LA SEQUENCE:
      (A) LONGUEUR: 16 acides aminés
      (B) TYPE: acide aminé
      (C) NOMBRE DE BRINS:
      (D) CONFIGURATION: linéaire
   (ii) TYPE DE MOLECULE: peptide
   (xi) DESCRIPTION DE LA SEQUENCE: SEQ ID NO: 1:
   Arg Gln Ile Lys Ile Trp Phe Gln Asn Arg Arg Met Lys Trp Lys Lys
   1               5                   10                  15
(2) INFORMATIONS POUR LA SEQ ID NO: 2:
   (i) CARACTERISTIQUES DE LA SEQUENCE:
      (A) LONGUEUR: 15 acides aminés
      (B) TYPE: acide aminé
      (C) NOMBRE DE BRINS:
      (D) CONFIGURATION: linéaire
   (ii) TYPE DE MOLECULE: peptide
   (xi) DESCRIPTION DE LA SEQUENCE: SEQ ID NO: 2:
   Thr Glu Arg Gln Ile Lys Ile Trp Phe Gln Asn Arg Arg Met Lys
   1               5                   10                  15
(2) INFORMATIONS POUR LA SEQ ID NO: 3:
   (i) CARACTERISTIQUES DE LA SEQUENCE:
      (A) LONGUEUR: 16 acides aminés
      (B) TYPE: acide aminé
      (C) NOMBRE DE BRINS:
      (D) CONFIGURATION: linéaire
   (ii) TYPE DE MOLECULE: peptide
   (xi) DESCRIPTION DE LA SEQUENCE: SEQ ID NO: 3:
   Lys Lys Trp Lys Met Arg Arg Asn Gln Phe Trp Ile Lys Ile Gln Arg
   1               5                   10                  15
(2) INFORMATIONS POUR LA SEQ ID NO: 4:
   (i) CARACTERISTIQUES DE LA SEQUENCE:
      (A) LONGUEUR: 16 acides aminés
      (B) TYPE: acide aminé
      (C) NOMBRE DE BRINS:
      (D) CONFIGURATION: linéaire
   (ii) TYPE DE MOLECULE: peptide
   (ix) CARACTERISTIQUE:
      (A) NOM/CLE: Peptide
      (B) EMPLACEMENT:1..16
      (D) AUTRES INFORMATIONS:/product= "Acides amines de la serie D"
   (xi) DESCRIPTION DE LA SEQUENCE: SEQ ID NO: 4:
   Arg Gln Ile Lys Ile Trp Phe Gln Asn Arg Arg Met Lys Trp Lys Lys
   1               5                   10                  15
(2) INFORMATIONS POUR LA SEQ ID NO: 5:
   (i) CARACTERISTIQUES DE LA SEQUENCE:
      (A) LONGUEUR: 16 acides aminés
      (B) TYPE: acide aminé
      (C) NOMBRE DE BRINS:
      (D) CONFIGURATION: linéaire
   (ii) TYPE DE MOLECULE: peptide
   (xi) DESCRIPTION DE LA SEQUENCE: SEQ ID NO: 5:
   Arg Gln Ile Lys Ile Trp Phe Pro Asn Arg Arg Met Lys Trp Lys Lys
   1               5                   10                  15
(2) INFORMATIONS POUR LA SEQ ID NO: 6:
   (i) CARACTERISTIQUES DE LA SEQUENCE:
      (A) LONGUEUR: 16 acides aminés
      (B) TYPE: acide aminé
      (C) NOMBRE DE BRINS:
      (D) CONFIGURATION: linéaire
   (ii) TYPE DE MOLECULE: peptide
   (xi) DESCRIPTION DE LA SEQUENCE: SEQ ID NO: 6:
   Arg Gln Pro Lys Ile Trp Phe Pro Asn Arg Arg Lys Pro Trp Lys Lys
   1 5 10 15
(2) INFORMATIONS POUR LA SEQ ID NO: 7:
   (i) CARACTERISTIQUES DE LA SEQUENCE:
      (A) LONGUEUR: 16 acides aminés
      (B) TYPE: acide aminé
      (C) NOMBRE DE BRINS:
      (D) CONFIGURATION: linéaire
   (ii) TYPE DE MOLECULE: peptide
   (xi) DESCRIPTION DE LA SEQUENCE: SEQ ID NO: 7:
   Arg Gln Ile Lys Ile Trp Phe Gln Asn Met Arg Arg Lys Trp Lys Lys
   1 5 10 15
(2) INFORMATIONS POUR LA SEQ ID NO: 8:
   (i) CARACTERISTIQUES DE LA SEQUENCE:
      (A) LONGUEUR: 16 acides aminés
      (B) TYPE: acide aminé
      (C) NOMBRE DE BRINS:
      (D) CONFIGURATION: linéaire
   (ii) TYPE DE MOLECULE: peptide
   (xi) DESCRIPTION DE LA SEQUENCE: SEQ ID NO: 8:
   Arg Gln Ile Lys Ile Trp Phe Gln Asn Arg Arg Met Arg Trp Arg Arg
   1 5 10 15
(2) INFORMATIONS POUR LA SEQ ID NO: 9:
   (i) CARACTERISTIQUES DE LA SEQUENCE:
      (A) LONGUEUR: 16 acides aminés
      (B) TYPE: acide aminé
      (C) NOMBRE DE BRINS:
      (D) CONFIGURATION: linéaire
   (ii) TYPE DE MOLECULE: peptide
   (xi) DESCRIPTION DE LA SEQUENCE: SEQ ID NO: 9:
   Arg Arg Trp Arg Arg Trp Trp Arg Arg Trp Trp Arg Arg Trp Arg Arg
   1 5 10 15
(2) INFORMATIONS POUR LA SEQ ID NO: 10:
   (i) CARACTERISTIQUES DE LA SEQUENCE:
      (A) LONGUEUR: 15 acides aminés
      (B) TYPE: acide aminé
      (C) NOMBRE DE BRINS:
      (D) CONFIGURATION: linéaire
   (ii) TYPE DE MOLECULE: peptide
   (xi) DESCRIPTION DE LA SEQUENCE: SEQ ID NO: 10:
   Arg Leu Arg Arg Leu Leu Arg Arg Leu Leu Arg Arg Leu Arg Arg
   1               5                   10                  15